# EUROPÄISCHE PATENTSCHRIFT

(11) **EP 1 523 298 B1**
(45) Veröffentlichungstag und Bekanntmachung des Hinweises auf die Patenterteilung: **31.12.2008**
(21) Anmeldenummer: 03787706.5
(22) Anmeldetag: 22.07.2003
(51) Int. Cl.: A61Q 5/00, A61Q 17/04, A61K 8/06, A61K 8/37, A61K 8/40, A61K 8/46, A61K 8/49, A61K 8/67

(54) **MIKROEMULSION ENTHALTEND UV-LICHTSCHUTZFILTER UND/ODER ANTISCHUPPENMITTEL**
MICROEMULSION CONTAINING ANTI-UV FILTERS AND/OR ANTI-DANDRUFF AGENTS
MICROEMULSION CONTENANT DES FILTRES ANTI-UV ET/OU DES AGENTS ANTIPELLICULAIRES

(30) Priorität: 22.07.2002 DE 10233330
(43) Veröffentlichungstag der Anmeldung: 20.04.2005
(73) Patentinhaber: SASOL Germany GmbH, 20537 Hamburg (DE)
(72) Erfinder: BROCK, Michael, 46514 Schermbeck (DE); HANNING, Thorsten, 48249 Dülmen (DE); KOBERSTEIN, Eva-Maria, 45657 Recklinghausen (DE); NAPIERALA, Heinz, 45699 Herten (DE)
(74) Vertreter: Schupfner, Georg
(86) Internationale Anmeldenummer: PCT/DE2003/002454
(87) Internationale Veröffentlichungsnummer: WO 2004/016233

(56) Entgegenhaltungen:
- EP-A- 0 374 414
- WO-A-00/47166
- WO-A-01/45615
- WO-A-99/09943
- WO-A-02/053695

## Beschreibung

Die Erfindung betrifft Mikroemulsionen enthaltend UV-Lichtschutzfilter und/oder Antischuppenmittel und deren Verwendung.

Die Pigmentierung normaler Haut führt unter dem Einfluss von Sonnenstrahlung zur Bildung von Melaninen. Dabei ruft die Bestrahlung mit UV-A Licht die Dunkelung der in der Epidermis vorhandenen Melaninkörper hervor, während UV-B Strahlung die Bildung des Melanins bewirkt. Bei zu intensivem und langanhaltendem Einwirken von Sonnenstrahlung auf die Haut erfolgt die Exposition mit UV-B Strahlung jedoch in einem Ausmaß, dass nicht ausreichend schnell Pigment gebildet werden kann. Dieses kann zu Hautrötungen (Erythemen) oder Hautentzündungen (Sonnenbrand) ggf. sogar zu Brandblasen führen. Um diese unerwünschten Nebenwirkungen zumindest zu mildern, wird eine große Anzahl von Sonnenschutzmitteln in verschiedenen Darreichungsformen angeboten. Dies sind im wesentlichen Cremes, Lotionen oder Sprays, die kurz vor oder während des intensiveren Einwirkens von Sonnenlicht auf die Haut aufgetragen werden. Dabei handelt es sich ganz überwiegend um milchig-trübe Makroemulsionen, die neben einigen Pflegestoffen vor allem UV-Lichtschutzfilter enthalten.

Die marktgängigen Sonnenschutzmittel tragen nicht dem zunehmenden Wunsch der Verbraucher nach kosmetischen Produkten mit multifunktionellen Eigenschaften Rechnung. So wünscht sich der Verbraucher kosmetische Produkte, die bei einmaliger Applikation mehrere Funktionen wie z.B. die Reinigung und Pflege der Haut zugleich verfüllen. Weiterhin wünscht der Verbraucher transparente Produkte, die dem zunehmenden Anspruch nach ästhetisch annehmbaren Formulierungen erfüllen. Somit besteht das Bedürfnis nach Produkten, die in einem Anwendungsschritt die Haut sowohl reinigen, als auch mit pflegenden Substanzen versorgen und auf der Haut einen Sonnenschutz bewirken.

Flüssige Zusammensetzungen, die als Körperreinigungs- und gleichzeitig als Körperpflegemittel Verwendung finden sollen, müssen verschiedensten Ansprüchen genügen. Sie sollen z.B. die reinigenden Eigenschaften einer wäßrigen Tensidformulierung mit den pflegenden Eigenschaften einer fettenden Ölkomponente vereinen. Die Reinigung der Haut und des Haars wird in der Regel von Tensiden übernommen. Je nach Art der verwendeten Tenside bewirken diese eine mehr oder weniger stark ausgeprägte Quellung und nachfolgendes Austrocknen der Homschicht der Haut, wodurch der Schutzmechanismus der Hautoberfläche gestört wird. Daher werden den bekannten Mitteln zur Reinigung der Haut in zunehmenden Maß hautpflegende Komponenten zur Hautregeneration zugesetzt. Die Art der Ölkomponente und deren Anteil in der späteren Formulierung sind ebenso wie der Anteil der wäßrigen Phase und deren Zusammensetzung häufig durch die Erfordernisse des Anwendungsbereiches vorgegeben. Märktgängige Zusammensetzungen, die diesen Ansprüchen genügen, liegen in der Regel als Makroemulsionen vor. Diese sind trüb und thermodynamisch instabil, d.h. sie separieren nach einiger Zeit irreversibel. Als Alternative bieten sich Mikroemulsionen an, die ästhetisch vorteilhaft optisch transparent und thermodynamisch stabil und somit auch lagerstabil sind.

Während dem Fachmann die Auswahl eines geeigneten Tensides oder einer geeigneten Tensidkombination zur Herstellung einer Makroemulsion aus der breiten Palette der am Markt befindlichen Tenside keine Schwierigkeit bereitet, stellt die Herstellung einer Mikroemulsion ein deutlich größeres Problem dar. Grund hierfür ist, dass die Phasengebiete einer Öl/Wasser/Tensid-Mischung, bei denen sich eine Makroemulsion ausbildet, deutlich größer sind als die Phasengebiete, bei denen eine Mikroemulsion vorliegt. Zu dem kann es je nach Anwendungsbereich gewünscht sein, dass sich weitere funktionelle Hilfsstoffe in die Mikroemulsion einarbeiten lassen, ohne dass die Stabilität der Mikroemulsion beeinträchtigt wird. Weiterhin sollen häufig sowohl wasser- als auch öllösliche Komponenten eingearbeitet werden, ohne dass die Mikroemulsion instabil wird.

Bekannte funktionelle Hilfsstoffe, deren Einsatz in Mikroemulsionen gewünscht ist, sind beispielsweise UV-Lichtschutzfilter zur Herstellung von reinigenden und pflegenden Zubereitungen mit Sonnenschutz und Antischuppenmittel, die zur Herstellung von Antischuppenhaarwaschmitteln geeignet sind.

Aufgabe der vorliegenden Erfindung war es, Formulierungen von kosmetischen und medizinisch-dermatologischen Mikroemulsionen zu schaffen, die es erlauben, sowohl öllösliche als auch wasserlösliche Lichtschutzfilter oder Antischuppenmittel einzuarbeiten, ohne die Stabilität der Mikroemulsion zu beeinträchtigen, die insbesondere geeignet sind zur Reinigung und Pflege der Haut.

Die Aufgabe wird erfindungsgemäß gelöst durch Mikroemulsionen enthaltend zumindest die folgenden Komponenten:
(A) 0,5 bis 70 Gew.-% Alkanolammonium-Salze der Alkylsulfate und/oder Alkylpolyalkylenglykolethersulfate der Struktur:

   R¹-O-(CₚH₂ₚO)ₘ-SO₃-HN⁺R²R³R⁴

   worin
   - R¹ =: ein C₈-bis C₂₀-Kohlenwasserstoffrest ist,
   - p =: eine ganze Zahl von 2 bis 5 ist, wobei p für jedes m verschieden sein kann,
   - R³ =: H, ein C₁- bis C₆-Alkyl oder ein C₂- bis C₄- Hydroxyalkyl, insbesondere Hydroxyisopropyl
   - R³ =: H, ein C₁- bis C₆- Alkyl oder ein C₂- bis C₄- Hydroxyalkyl, insbesondere Hydroxyisopropyl,
   - R⁴ =: ein C₂- bis C₄- Hydroxyalkyl, insbesondere Hydroxyisopropyl und
   - m =: eine ganze Zahl von 0 bis 7 ist,
   oder deren Gemische,
(B) 20 bis 95 Gew.-% Wasser,
(C) 0,1 bis 20 Gew.-% einer oder mehrerer Ölkomponenten und
(D) 0,1 bis 20 Gew.-% eines oder mehrerer ein- oder mehrwertige C₂- bis C₂₄-Alkohole, sowie
(E.1) 0,1 bis 15 Gew.-% eines oder mehrerer UV-Lichtschutzfilter und/oder
(E.2) 0,1 bis 3 Gew.-% eines oder mehrerer Antischuppenmittel,
jeweils bezogen auf die Gesamtzusammensetzung.

Bevorzugte Ausführungsformen oben genannter Zusammensetzung sind nachfolgend beschrieben bzw. Gegenstand der Unteransprüche. Weiterhin beansprucht ist die Verwendung der Mikroemulsionen im kosmetischen Bereich und/oder zur Herstellung eines Arzneimittels zur Verwendung im medizinisch-dermatologischen Bereich, insbesondere als Sonnenschutzmittel, als Schaum zur Aufbringung mittels handbetriebener Pumpschäumer ohne Verwendung von Treibgasen, als mit Wasser abzuspülendes Reinigungs- und Pflegeprodukt und als Antischuppenshampoo.

Mikroemulsionen enthaltend Alkanolammonium-Salze der Alkylsulfate und/oder Alkylpolyalkylenglykolethersulfate sind aus der WO 00/47166-A2 bekannt. Die in der WO 00/47166-A2 offenbarten Mikroemulsionen werden hiermit ausdrücklich auch zum Gegenstand dieser Anmeldung gemacht, um im Wege der Verweisung Bestandteil der Offenbarung des vorliegenden Textes zu werden.

Die erfindungsgemäßen Mikroemulsionen können (fakultativ) weiterhin unabhängig voneinander zumindest eine der folgenden Komponenten enthalten:
(F) größer 0 bis 20 Gew.-%, vorzugsweise 3 bis 15 Gew.-%, eines oder mehrerer weiterer Tenside,
(G) größer 0 bis 20 Gew.-%, vorzugsweise 1 bis 15 Gew.-% oder auch 2 bis 10 Gew.%, eines oder mehrerer Elektrolyte und/oder
(H) größer 0 bis 10 Gew.-%, vorzugsweise 0,1 bis 8 Gew.-%, eines oder mehrerer Additive.

Die Mikroemulsionen weisen besonders vorteilhaft und unabhängig voneinander die oben genannten Komponenten in den unten angegebenen Konzentrationen auf
(A) zu 2 bis 60 Gew.-%, vorzugsweise 5 bis 40 Ges.-%,
(B) zu 30 bis 80 Gew.-%, vorzugsweise 40 bis 60 Gew.-%,
(C) zu 0,5 bis 15 Gew.-%, vorzugsweise 1 bis 10 Gew.-% und
(D) zu 0,1 bis 9 Gew.%, vorzugsweise 0,5 bis 9 Gew.-%, sowie
(E.1) zu 0,5 bis 10 Gew.-%, vorzugsweise 1 bis 8 Gew.-% und/oder
(E.2) zu 0,2 bis 2 Gew.-%, vorzugsweise 0,3 bis 1,5 Gew.-%, und ggf.:
(F) größer 0 bis 20 Gew.-%, vorzugsweise 3 bis 15 Gew.-%, weitere Tenside,
(G) größer 0 bis 20 Gew.-%, vorzugsweise 1 bis 12 Gew.-%, Elektrolyte und/oder
(H) größer 0 bis 10 Gew.-%, vorzugsweise 0,1 bis 8 Gew.-% Additive,
worin weiterhin besonders vorteilhaft
(F) als weiteres Tensid zu zumindest 1 Gew.% ein mit Ethylenoxid und/oder Propylenoxid oxalkyliertes und anschließend mit C6- bis C22- Fettsäuren teilweise oder ganz verestertes Triglycerid ist bzw. enthält, vorzugsweise zu 1 bis 20 Gew.%.

Die erfindungsgemäßen Mikroemulsionen sind im Gegensatz zu Makroemulsionen thermodynamisch stabile, optisch transparente und makroskopisch homogene Mischungen aus zwei nicht miteinander mischbaren Flüssigkeiten, nämlich von Wasser (B) und einer Ölkomponente (C), denen die unter (A) erwähnten Tensidmoleküle zugesetzt wurden. Die erfindungsgemäßen Mikroemulsionen sind z.B. bei Temperaturen von 15 bis 80°C, vorzugsweise unter 55°C, herstellbar und zumindest bis zu einer Temperatur von 60 °C stabil. Die mittlere Teilchengröße der dispersen Phase beträgt vorzugsweise weniger als 100 nm.

Die beanspruchten Mikroemulsionen weisen in der Regel über einen breiten Zusammensetzungsbereich keine Bildung von flüssigkristallinen Phasen auf. Vorteilhaft finden die beanspruchten Mikroemulsionen Verwendung im kosmetischen und/oder medizinisch-dermatologischen Bereich. Besonders bevorzugt werden die erfindungsgemäßen Mikroemulsionen als oder in Körperreinigungs- und Körperpflegemitteln eingesetzt.

Besonders vorteilhaft enthalten die oben beschriebenen erfindungsgemäßen Zusammensetzungen Alkanolammoniumsalze der Alkylsulfate und/oder Alkylpolyalkylenglykolethersulfate der oben beschriebenen allgemeinen Struktur, die unabhängig voneinander vorzugsweise die folgenden Reste aufweisen:
- R¹ =: C₁₂₋ bis C₁₆- Alkyl, wobei der Alkylrest linear und gesättigt ist,
- p =: 2 oder 3 ist, wobei p für jedes m verschieden sein kann,
- R² =: H oder Hydroxyisopropyl,
- R³ =: H oder Hydroxyisopropyl,
- R⁴ =: Hydroxyisopropyl und
- m =: 0,1 oder 2 ist.

Nachfolgend werden vorteilhafte Ausführungsformen der Erfindung in Bezug auf die Komponenten (C) bis (H) erläutert.

### Ölkomponente (C)

Vorteilhaft werden erfindungsgemäße Ölkomponenten aus der Gruppe der Lecithine und der Mono-, Di- und /oder Triglyceride gesättigter und/oder ungesättigter, verzweigter und/oder linearer Alkylcarbonsäuren einer Kettenlänge von 8 bis 24, insbesondere 12 bis 18 C-Atomen, eingesetzt. Die Fettsäuretriglyceride können vorteilhaft synthetische, halbsynthetische oder natürliche Öle sein, wie z.B. Sojaöl, Rizinusöl, Olivenöl, Safloröl, Weizenkeimöl, Traubenkernöl, Sonnenblumenöl, Erdnußöl, Mandelöl, Palmöl, Kokosöl, Distelöl, Nachtkerzenöl, Rapsöl und dergleichen.

Weiterhin kann die Ölkomponente Vaseline, Paraffinöl und Polyolefine enthalten oder aus diesen bestehen. Die Ölkomponenten können im Sinne der vorliegenden Erfindung ferner vorteilhaft gewählt werden aus der Gruppe der Ester aus gesättigten und/oder ungesättigten, verzweigten und/oder linearen Alkylcarbonsäuren einer Kettenlänge von 3 bis 30 C-Atomen und gesättigten und/oder ungesättigten, verzweigten und/oder linearen Alkoholen einer Kettenlänge von 3 bis 30 C-Atomen sowie aus der Gruppe der Ester aus aromatischen Carbonsäuren und gesättigten und/oder ungesättigten, verzweigten und/oder linearen Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropylleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyl-decylstearat, 2-Octyldodecylpalmitat, Oleyloleat, Oleylerucat, Erucyloleat, Erucylerucat, sowie synthetische, halbsynthetische und natürliche Gemische solcher Ester, wie z.B. Jojobaöl.

Ferner kann die Ölkomponente vorteilhaft gewählt werden aus der Gruppe der verzweigten und linearen Kohlenwasserstoffe und -wachse und der Silikonöle. Auch beliebige Mischungen der vorgenannten Ölkomponenten sind vorteilhaft im Sinne der Erfindung.

### Alkohole (D)

Die beanspruchten Mikroemulsionen enthalten ein- oder mehrwertige, vorzugsweise ein-, zwei- oder dreiwertige, C₂- bis C₂₄- Alkohole, vorzugsweise gesättigte und/oder verzweigte und/oder lineare Alkohole. Beispielhaft seien genannt: Ethanol, Propanol, Isopropanol, Butanol, Pentanol, Hexanol, Heptanol, Oktanol, 2-Ethylhexanol, Laurylalkohol, Myristolalkohol, Palmitylalkohol, Sterylalkohol, Oleylalkohol, Elaidylalkohol, Guerbetalkohole und Alkylenglykole, wie z.B. Ethylenglykol, Propylenglykol und Glycerin. Besonders bevorzugt ist Propylenglykol.

### Lichtschutzfilter (E.1)

Überraschender Weise ist es möglich, verschiedene sowohl wasserlösliche als auch öllösliche UV-Lichtschutzfilter stabil in die Mikroemulsionen einzuarbeiten.

Die UV-Lichtschutzfilter enthaltenden Mikroemulsionen können vorteilhaft zur Reinigung und Pflege der Haut verwendet werden, wobei der UV-Lichtschutzfilter nach Applikation auf der Haut verbleibt und einen Sonnenschutz bewirkt. Somit ist die Behandlung der Haut nach dem Waschen mit Sonnenschutzmitteln nicht mehr notwendig.

Unter UV-Lichtschutzfiltern im Sinne der Erfindung sind organische Substanzen zu verstehen, die in der Lage sind, ultraviolette Strahlen zu absorbieren und die aufgenommene Energie in Form längerwelliger Strahlung, z. B. Wärme wieder abzugeben. Es wird zwischen UVA-Filtern und UVB-Filtern unterschieden, je nachdem, welcher Wellenbereich umgewandelt wird. UVA-Filter dienen der Filterung der langwelligeren UVA-Strahlen (320 - 400 nm), während UVB-Filter die kurzwelligeren UVB-Strahlen (295 - 320 nm) abhalten sollen.

Als öllösliche UV-Lichtschutzfilter Substanzen sind z.B. zu nennen:
- 3-Benzylidencampher und dessen Derivate, z.B. 3-(4-Methylbenzyliden)-campfer;
- 4-Aminobenzoesäurederivate, z.B. 4-(Dimethylamino)benzoesäure-2-ethylhexylester, 4-(Dimethylamino)benzoesäure-2-octylester oder 4-(Dimethylamino)benzoesäureamylester;
- Ester der Zimtsäure, z.B. 4-Methoxyzimtsäure-2-ethylhexylester, 4-Methoxyzimtsäureisopentylester, 2-Cyano-3-phenylzimtsäure-2-ethylhexylester (Octocrylene);
- Ester der Salicylsäure, z.B. Salicylsäure-2-ethylhexylester, Salicylsäure-4-- isopropylbenzylester, Salicylsäure-homomenthylester;
- Derivate des Benzophenons, z.B. 2-Hydroxy-4-methoxybenzophenon, 2-Hydroxy-4-methoxy-4-methylbenzophenon, 2,2'-Dihydroxy-4-methoxy-benzophenone;
- Ester der Benzalmalonsäure, z.B. 4-Methoxybenzmalonsäuredi-2-ethylhexylester;
- Triazinderivate, z.B. 2,4,6 -Trianilino-(p-carbo-2'-ethyl-1'-hexyloxy)-1,3,5-triazin, Octyltriazon;
- Propan-1,3-dione, z.B.1-(4-tert.-butylphenyl)-3-(4'-methoxyphenyl)propan-1,3-dion.

Als wasserlösliche Substanzen sind z. B. zu nennen:
- Phenylbenzimidazolsulfonsäure und deren Salze, z.B. Alkali-, Erdalkali-, Ammonium-, Alkylammonium-, Alkanolammonium- und Glucammoniumsalze;
- Sulfonsäurederivate von Benzophenon, z.B. 2-Hydroxy-4-methoxybenzophenon-5-sulfonsäure und ihre Salze;
- Sulfonsäurederivate des 3-Benzylidencamphers, z. B. 4-(2-Oxo-3-bornylidenmethyl) benzolsulfonsäure und 2-Methyl-5-(2-Oxo-3-bornyliden)-sulfonsäure und deren Salze;
- Derivate der 4-Aminobenzoesäure, z.B. 4-Bis(polyethoxy)paraaminobenzoesäurepolyethoxyethylester.

Als typische UV-Lichtschutzfilter kommen insbesondere Octocrylene, 4-Methoxyzimtsäure-2-ethylhexylester, 2-Phenylbenzimidazol-5-sulfonsäure, 2-Hydroxy-4-methoxybenzophenonsulfonsäure und 4-Bis(polyethoxy)paraaminobenzoesäure polyethoxyethylester sowie deren Mischungen in Frage.

Neben den genannten löslichen Stoffen kommen auch unlösliche Pigmente, nämlich feindisperse Metalloxide bzw. Salze als UV-Lichtschutzfilter in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen.

Neben den genannten Gruppen primärer Lichtschutzstoffe können auch sekundäre Lichtschutzmittel vom Typ der Antioxidantien eingesetzt werden, die die photo-chemische Reaktionskette unterbrechen, welche ausgelöst wird, wenn UM-Strahlung in die Haut eindringt. Typische Beispiele hierfür sind Tocopherole und Ascorbinsäure und ihre Ester.

### Antischuppenmittel (E.2)

Überraschenderweise ist die Einarbeitung wasserlöslicher ebenso wie öllösliche Antischuppenmittel in die Mikroemulsionen problemlos möglich.

Die Antischuppenmittel enthaltenden Mikroemulsionen können vorteilhaft als Antischuppenshampoo zur Reinigung und Pflege des Haars verwendet werden. Neben der Minderung der Schuppenbildung üben die in den Mikroemulsionen enthaltenen Öle einen pflegenden Einfluss auf die Kopfhaut aus.

Unter Antischuppenmittel sind Substanzen zu verstehen, die neben ihrer spezifischen proliferationshemmenden Wirkung einen keratolytischen Effekt besitzen. Sie entfernen die Schuppen und Mikroorganismen der Kopfhaut. Der Wirkungsmechanismus der Antischuppenmittel besteht in der Normalisierung der bei Kopfschuppen deutlich erhöhten Zellteilungsaktivität in der Epidermis. Daneben haben die Antischuppenmittel eine antimikrobielle Wirkung.

Als Antischuppenmittel können 1-Hydroxy-4-methyl-6-(2,4,4-trimythylpentyl)-2-(1H)-pyridon-monoethanolaminsalz), 1-Acetyl-4-[4-[[2-(2,4-dichlorophenyl)-2-(1H-imidazol-1-ylmethyl)-1,3-dioxolan-4-yl]methoxy]phenyl]piperazin, Selendisulfid, Schwefel kolloidal, Schwefelpolyethylenglykolsorbitanmonooleat, Schwefelrizinolpolyethoxylat, Schwefelteer Destillate, Salicylsäure (bzw. in Kombination mit Hexachlorophen), Undecylensäure Monoethanolamid, Sulfosuccinat Na-Salz, Kalium-Salz des Kondensationsproduktes von Undecylensäurechlorid und hydrolysiertem Collagen, Zinkpyrethion, Aluminiumpyrition und Magnesiumpyrithion/Dipyrithion-Magnesiumsulfat eingesetzt werden.

Besonders bevorzugte öllösliche Antischuppenmittel im Sinne der Erfindung sind 1-(4-Chlorophenoxy- 1 -(1H-imidazol-1-yl)-3, 3-dimethyl-2-butanon (Climbazol) und 3-Aminopyridin (Niacinamid).

Besonders bevorzugte wasserlösliche Antischuppenmittel im Sinne der Erfindung sind die Verbindung von 2-Aminoethanol mit 1-Hydroxy=4-methyl-6-(2,4,4-trimethylpentyl)-2(1 H)-pyridon (1:1) (Piroctone Olamine).

### Weitere Tenside bzw. Emulgatoren (F)

Neben den genannten Alkanolammoniumsalzen der Alkylsulfate und/oder Alkylpolyalkylenglykolethersulfate können die erfindungsgemäßen Mikroemulsionen weitere Tenside enthalten. Vorteilhaft sind dies ein oder mehrere Tenside aus der Gruppe:
- Alkoholpolyethylenglykolether, z.B. solchen der allgemeinen Formel R-O-(C₂H₄O)ₙ-H, wobei R einen verzweigten oder linearen, gesättigten oder ungesättigten C₈- bis C₂₀- Alkylrest und n eine Zahl von 2 bis 20 darstellen, Fettsäureesterpolyethylenglykolether, z.B. solchen der allgemeinen Formel R-COO-(C₂H₄O)p-H; wobei R einen verzweigten oder linearen, gesättigten oder ungesättigten C₇- bis C₁₉- Alkylrest und p eine Zahl von 2 bis 40 darstellen,
- Alkylpolyalkylenglykolethercarbonsäuren, z.B. solchen der allgemeinen Formel R-O-(C₂H₄O)ₙ-CH₂-COOH bzw. deren Alkanolammonium-oder Alkalimetallsalze , wobei R einen verzweigten oder linearen, gesättigten oder ungesättigten C₈- bis C₂₀- Alkylrest und n eine Zahl von 2 bis 20 darstellen,
- Alkylamidoalkylbetaine, z.B. solchen der allgemeinen Formel R-CONH(CH₂)ᵤN⁺(CH₃)₂- CH₂-COO⁻, wobei R einen verzweigten oder linearen, gesättigten oder ungesättigten C₇- bis C₁₉- Alkylrest und u eine Zahl von 1 bis 10 darstellen,
- Produkte aus der Alkoxylierung von Triglyceriden, die ganz oder teilweise mit C₆- bis C₂₂ Fettsäuren verestert sind, wobei pro Mol Triglycerid 2 bis 40 Mol Alkoxylierungsmittel eingesetzt werden (als besonders bevorzugte Tensidgruppe), z.B. mit Ölsäure teilveresterte Anlagerungsprodukte von Rizinusöl und/oder gehärtetem Rizinusöl mit Ethylenöxid.
- Partiell neutralisierte Partialglyceride von ein oder mehrwertigen C2- bis C22-Carbonsäuren, wie z.B. Linolsäure, Stearinsäure, Isostearinsäure, Palmitinsäure, Laurinsäure, Caprylsäure, Caprinsäure, Citronensäure und/oder Milchsäure.
- Ester des Polyglycerins, worin die Carbonsäure-Gruppe vorzugsweise 2 bis 22 Kohlenstoffatome aufweist.

Vorzugsweise sind in der erfindungsgemäßen Zusammensetzung weiterhin keine oder allenfalls sehr geringe Anteile (kleiner 0,5 Gew.-%) anionischer Tenside des Sulphonat-Typs enthalten und weiterhin sind vorzugsweise in der erfindungsgemäßen Zusammensetzung nur sehr geringe Anteile (kleiner 0,5 Gew.-%) Fettsäurepolyglyholestersulfate enthalten und insbesondere keine Fettsäurepolyglykolestersulfate.

### Elektrolyte (G)

Die erfindungsgemäßen Mikroemulsionen können Elektrolyte enthalten. Beispielhaft seien Alkali- und Erdalkalisalze, wie z.B. die entsprechenden Halogenide, Sulfate, Phosphate oder Citrate genannt.

### Additive (H)

Additive sind beispielhaft Poly(C₂- bis C₄- )alkylenglykole, insbesondere Polyethylenglykole und/oder Polypropylenglykole, jeweils vorzugsweise mit einem Molgewicht bis 1500 g/mol, Parfüme, Farbstoffe, Hydrotropica, Verdicker, Perlglanzagenzien, Eiweißhydrolysate, Pflanzenextrakte, Vitamine, α-Hydroxycarbonsäuren und ihre Ester, antimokrobielle Stoffe und dergleichen. Die Lichtschutzfilter (E:1) und die Antischuppenmittel (E.2) werden im Sinne der Erfindung nicht als Additive (H) verstanden.

Die erfindungsgemäßen Zusammensetzungen sind insbesondere zur Herstellung von Schäumen durch eine mit Fingerdruck betriebenen Pumpschäumer geeignet (nicht unter Anwendung eines unter Druck stehenden Treibmittels), wie sie z.B. Airspray International anbietet.

Die nachfolgenden Beispiele, sollen die vorliegende Erfindung verdeutlichen, ohne sie einzuschränken. Die Zahlenwerte in den Beispielen stehen für Gewichtsprozente, bezogen auf das Gesamtgewicht der jeweiligen Mikroemulsionen.

### Beispiel 1

| | | |
|---|---|---|
| a) | MARLINAT^{®} 242/90 M | 15,5 Gew.-% |
| | MARLOWET^{®} LVS | 3,9 Gew.-% |
| | Avocadoöl | 0,7 Gew.-% |
| | Jojobaöl | 0,2 Gew.-%. |
| | MARLINAT^{®}CM 105/80 | 1,9 Gew.-% |
| b) | Neo Heliopan^{®} Hydro* | 4,0 Gew.-% |
| | VE-Wasser | 63,6 Gew.-% |
| | NaOH, 20 %ig | 2,6 Gew.-% |
| | NaCl | 1,9 Gew.-% |
| | Ampholyt JB 130 K | 2,7 Gew.% |
| c) | Antil^{®}141, liquid | 1,0 Gew.-% |
| | Laurylglucosid | 1,0 Ges.-% |
| | Parfüm | 1,0 Gew.-% |
| | Konservierungsmittel | q.s. |

| | | |
|---|---|---|
| * mit 20 %iger NaOH auf pH =6,5 eingestellt | | |

Herstellung: Die gesamte Herstellung erfolgte bei 20 °C. Die unter a) und b) aufgeführten Komponenten wurden separat zusammengegeben und homogen gerührt. Anschließend wurde Phase b) langsam bei ständigem Rühren zu Phase a) gegeben.

In die vereinigte klare Phase wurden abschließend die unter c) angegebenen Komponenten bei ständigem Rühren zugegeben. Es entstand ein klares Produkt mit hohem Schäumvermögen.

### Beispiel 2 (nicht gemäß der beanspruchten Erfindung)

| | | |
|---|---|---|
| a) | MARLINAT^{®} 242/90 M | 16,0 Gew.-% |
| | MARLOWET^{®} LVS | 4,0 Gew .-% |
| | Avocadoöl | 0,6 Ges.-% |
| | Jojobaöl | 0,2 Gew.-% |
| | MARLINAT^{®}CM 105/80 | 2,0 Gew.-% |
| b) | VE-Wasser | 65,4 Gew.-% |
| | Nach | 3,0 Gew.-% |
| | Ampholyt JB 130 K | 2,8 Gew.-% |
| c) | Antil^{®}141, liquid | 3,0 Gew.-% |
| | Parfüm | 1,0 Gew.-% |
| | Konservierungsmittel | q.s. |

### Herstellung: Die Herstellung erfolgte gemäß Beispiel 1

### Beispiel 3

| | | |
|---|---|---|
| a) | MARLINAT^{®} 242/90 M | 28,0 Gew.-% |
| | MARLOWET^{®} LVS | 7,0 Gew.-% |
| | LIPOXOL^{®} 600 | 2,0 Gew.-% |
| | Sojaöl | 4,0 Gew.% |
| | Rizinusöl | 1,0 Gew.-% |
| | MARLINAT^{®} CM 105/80 | 3,8 Gew.% |
| | Eiweißhydrolysat | 0,5 Gew.-% |
| | Uvinul^{®} MC 80 | 3,0 Gew.-% |
| b) | VE-Wasser | 38,7 Gew.-% |
| | NaCl | 2,0 Gew.-% |
| | Ampholyt JB 130 K | 5,0 Gew.-% |
| | Uvinul^{®} MS 40* | 2,0 Gew.-% |
| c) | Antil^{®}141, liquid | 2,0 Ges.-% |
| | Parfüm | 1,0 Gew.-% |
| | Konservierungsmittel | q.s. |

| | | |
|---|---|---|
| * mit 20 %iger NaOH auf pH = 6,5 eingestellt | | |

### Herstellung: Die Herstellung erfolgte gemäß Beispiel 1.

### Beispiel 4

| | | |
|---|---|---|
| a) | MARLINAT^{®} 242/90 M | 28,0 Gew.-% |
| | MARLOWET^{®} LVS | 7,0 Gew.-% |
| | LIPOXOL^{®} 600 | 2,0 Gew.-% |
| | Sojaöl | 4,0 Gew.-% |
| | Rizinusöl | 1,0 Gew.-% |
| | MARLINAT^{®} CM 105/80 | 3,8 Gew.-% |
| | Eiweißhydrolysat | 0,5 Gew.-% |
| | Uvinul^{®} MC 80 | 3,0 Gew.-% |
| b) | VE-Wasser | 39,7 Gew.-% |
| | NaCl | 1,0 Gew.-% |
| | Ampholyt JB 130 K | 5,0 Gew.-% |
| | Uvinul^{®} P 25 | 2,0 Gew.-% |
| c) | Antil^{®}141, liquid | 2,0 Gew.-% |
| | Parfüm | 1,0 Gew.-% |
| | Konservierungsmittel | q.s. |

### Herstellung: Die Herstellung erfolgte gemäß Beispiel 1

### Beispiel 5

| | | |
|---|---|---|
| a) | MARLINAT^{®} 242/90 M | 19,0 Gew.-% |
| | MARLOWET^{®} LVS | 2,5 Gew.-% |
| | RonaCare^{®} Nicotinamide | 0,7Gew.-% |
| | Jojobaöl | 1,0 Gew:-% |
| | MARLINAT^{®}CM 105/80 | 2,3 Gew.-% |
| b) | VE-Wasser | 68,6 Gew.-% |
| | NaCl | 2,4 Gew.-% |
| | Ampholyt JB 130K | 1,8 Gew.-% |
| c) | Antil^{®}141, liquid | 0,6 Gew.-% |
| | Parfüm | 0,6gen.-% |
| | Konservierungsmittel | q.s. |

### Herstellung: Die Herstellung erfolgte gemäß Beispiel 1

### Beispiel 6

| | | |
|---|---|---|
| a) | MARLINAT^{®} 242/90M | 15,0 Gew.-% |
| | MARLOWET^{®} LVS | 2,0 Gew.-% |
| | Crinipan^{®}AD | 0,7 Gew.-% |
| | Jojobaöl | 1,0 Gew.-% |
| | MARLINAT^{®} CM 105/80 | 1,8 Ges.-% |
| b) | VE-Wasser | 74,0 Gew.-% |
| | NaCl | 2,0 Gew.-% |
| | Ampholyt JB 130K | 1,5 Gew.-% |
| c) | Antil^{®} 141, liquid | 1,0 Gew.-% |
| | Parfüm | 0,5 Gew.-% |
| | Konservierungsmittel | q.s. |

### Herstellung: Die Herstellung erfolgte gemäß Beispiel 1

### Beispiel 7

| | | |
|---|---|---|
| a) | MARLINAT^{®} 242/90, M | 19,0 Gew.-% |
| | MARLOWET^{®} LVS | 2,5 Gew.-% |
| | Octopirox^{®} | 0,5 Gew.-% |
| | Jojobaöl | 1,0 Gew.-% |
| | MARLINAT^{®} CM 105/80 | 2,3 Gew.-% |
| b) | VE-Wässer | 67,3 Gew.-% |
| | NaCl | 2,4 Gew.-% |
| | Ampholyt JB 130 K | 1,8 Gew.-% |
| c) | Antil^{®} 141, liquid | 0,6Gew.-% |
| | Aloe Vera (1:1) | 2,0 Gew.-%. |
| | Parfüm | 0,6 Gew.-% |
| | Konservierungsmittel | q.s. |

### Herstellung: Die Herstellung erfolgte gemäß Beispiel 1

In den Beispielen 1 bis 7 wurden die folgenden Produkte eingesetzt

| | |
|---|---|
| MARLINAT^{®} 242/90 M: | C_{12/14}-Alkylpolyethylenglykol (2 EO)ethersulfat-Monoisopropanolammonium Salz, in 1,2-Propylenglykol (Sasol Germany GmbH) |
| MARLINAT^{®} CM 105/80: | C_{12/14}-Alkylpolyethylenglykol (10 EO)ethercarbonsäure-Natriumsalz, |
| | (Sasol Germany GmbH) |
| MARLOWET^{®} LVS: | Oxethyliertes Rizinusöl, teilverestert mit Ölsäure |
| | (Sasol Germany GmbH) |
| LIPOXOL^{®} 600: | Polyethylenglykol 600 (Sasol Germany GmbH) |
| Ampholyt JB 130 K: | Kokoamidopropyldimethylbetain |
| | (Sasol Germany GmbH) |
| Neo Heliopan^{®} Hydro: | 2-Phenylbenzimidazol-5-sulfonsäure |
| | (Haarmann & Reimer GmbH) |
| Neo Heliopan^{®} 303: | Octocrylene |
| | (Haarmann & Reimer GmbH) |
| Antil^{®} 141, liquid: | Polyethylenglykol-propylenglykol-dioleat |
| | (Degussa AG) |
| Uvinul^{®} MC 80: | 4-Methoxyzimtsäure-2-ethylhexylester |
| | (BASF AG) |
| Uvinul^{®} MS 40: | 2-Hydroxy-4-methoxybenzophenon-5-sulfonsäure |
| | (BASF AG) |
| Uvinul^{®} P 25: | 4-Bis(polyethoxy)paraminobenzoesäurepolyethoxyethylester (BASF AG) |
| RonaCare^{™}Nicotinamide: | Niacinamid (Merck KGaA) |
| Crinipan^{®} AD: | Climbazol (Haarmann & Reimer GmbH) |
| Octopyrox^{®}: | Piroctone Olamine (Clariant GmbH) |

Alle beispielhaft angegebenen Formulierungen zeichnen sich durch hohe Reinigungskraft, hohes Schäumvermögen, gutes Anschäumvermögen, Lagerstabilität und Häutmildheit aus.

## Patentansprüche

1. Mikroemulsion enthaltend zumindest die folgenden Komponenten:
(A) 0,5 bis 70 Gew.-% Alkanolammonium-Salze der Alkylsulfate und/oder Alkylpolyalkylenglykolethersulfate der Struktur:
R¹-O-(CₚH₂ₚO)ₘ-SO₃-HN⁺R²R³R⁴ ,
worin
R¹ = ein C₈- bis C₂₀- Kohlenwasserstoffrest ist,
p = eine ganze Zahl von 2 bis 5 ist, wobei p für jedes verschieden sein kann,
R² = H; ein C₁- bis C₆- Alkyl oder ein C₂- bis C₄- Hydroxyalkyl,
R³ = H, ein C₁- bis C₆- Alkyl oder ein C₂- bis C₄- Hydroxyalkyl,
R⁴ = ein C₂- bis C₄- Hydroxyalkyl und
m = eine ganze Zahl von 0 bis 7 ist,
oder deren Gemische,
(B) 20 bis 95 Gew.-% Wasser,
(C) 0,1 bis 20 Gew.-% eines oder mehrerer Ölkomponenten und
(D) 0,1 bis 20 Gew.-% eines oder mehrerer ein- oder mehrwertiger C₂- bis C₂₄-Alkohole, sowie
(E.1) 0,1 bis 15 Gew.-% eines oder mehrerer UV-Lichtschutzfilter und/oder
(E.2) 0,1 bis 3 Gew.-% eines oder mehrerer Antischuppenmittel,
jeweils bezogen auf die Gesamtzusammensetzung.

2. Mikroemulsion gemäß Anspruch 1, **dadurch gekennzeichnet, dass** der(die) UV-Lichtschutzfilter ausgewählt ist (sind) aus der Gruppe: 3-Benzylidencampher und dessen Derivate, 4-Aminobenzoesäurederivate, Ester der Zimtsäure, Ester der Salicylsäure, Derivate des Benzophenons, Ester der Benzalmalonsäure, Triazinderivate, Propan-1,3-dione, Phenylbenzimidaiolsulfonsäure und deren Salze, Sulfonsäurederivate von Benzophenon, Sulfonsäurederivate des 3-Benzylidencamphers, Derivate der 4-Aminobenzoesäure und feindisperse Metalloxide bzw. Salze.

3. Mikroemulsion gemäß einem der vorhergehenden Ansprüche, **dadurch gekennzeichnet, dass** der UV-Lichtschutzfilter (E.1) eine oder mehrere der folgenden Substanzen ist: Octocrylene, 4-Methöxyzimtsäure-2-ethylhexylester, 2-Phenylbenzimidazol-5-sulfonsäure, 2-Hydroxy-4-methoxybenzophenon-sulfonsäure und 4-Bis(polyethoxy)paraaminobenzoesäurepolyethoxyethylester sowie deren Mischungen.

4. Mikroemulsion gemäß Anspruch 1, **dadurch gekennzeichnet, dass** das Antischuppenmittel (E.2) eine oder mehrere der folgenden Substanzen ist: 1-(4-Chlorophenoxy-1-(1-H-imidazol-1-yl)-3,3-dimethyl-2-butanon, 3-Aminopyridin und die Verbindung von 2-Aminoethanol mit 1-Hydroxy-4-methyl-6-(2,4,4-trimethylpentyl)-2(1H)-pyridon.

5. Mikroemulsion gemäß Anspruch 1, worin die Alkanolammonium-Salze der Alkylsulfate und/oder Alkylpolyalkylenglykolethersulfate unabhängig voneinander folgende Reste bzw. Indizes aufweisen:
R¹ = ein linearer oder gesättigter C₁₂- bis C₁₆- Alkylrest,
p = 2 oder 3 ist, wobei p für jedes m verschieden sein kann,
R² = H oder Hydroxyisopropyl,
R³ = H oder Hydroxyisopropyl,
R⁴ = Hydroxyisopropyl und
m = eine ganze Zahl von 0 bis 2 ist.

6. Mikroemulsion gemäß einem der, vorhergehenden Ansprüche, wobei die Komponenten
(A) zu 2 bis 60 Gew.-% ,
(B) zu 30 bis 80 Gew.-%,
(C) zu 0,5 bis 15 Gew.-% und
(D) zu 0,1 bis 9 Gew.-%
in der Mikroemulsion enthalten sind.

7. Mikroemulsion gemäß einem der vorhergehenden Ansprüche, weiterhin enthaltend zumindest eine der folgenden Komponenten
(F) größer 0 bis 20 Gew.-% eines oder mehrerer weiterer Tenside bzw. Emulgatoren,
und ggf. auch
(G) größer 0 bis 20 Gew.-% eines oder mehrerer Elektrolyte und
(H) größer 0 bis 10 Gew.-% eines oder mehrerer Additive.

8. Mikroemulsion gemäß Anspruch 7, enthaltend zumindest folgende Komponente:
(F) zumindest 1 Gew.% eines Produktes aus der Alkoxylierung von Triglyceriden, das ganz oder teilweise mit C₆- bis C₂₂-Fettsäuren verestert ist, wobei vorzugsweise pro Mol Triglycerid 2 bis 40 Mol Alkoxylierungsmittel eingesetzt werden.

9. Mikroemulsion gemäß einem der vorhergehenden Ansprüche, wobei die Ölkomponente (C) eine oder mehrere Komponenten enthält ausgewählt aus der Gruppe der Lecithine, der Mono-, Dir- und /oder Triglyceride gesättigter und/oder ungesättigter, verzweigter und/oder linearer Carbonsäuren einer Kettenlänge von 8 bis 24 C-Atomen, verzweigten und/oder linearen Kohlenwasserstoffen, der Wachse; Vaseline, Paraffinöle, Polyolefine, Silikonöle und Ester aus gesättigten, ungesättigten und/oder aromatischen, verzweigten und/oder linearen Carbonsäuren einer Kettenlänge von 3 bis 30 C-Atomen und gesättigter und/oder ungesättigter, verzweigter und/oder linearer Alkohole einer Kettenlänge von 3 bis 30 C-Atomen.

10. Mikroemulsion gemäß einem der vorhergehenden Ansprüche, **dadurch gekennzeichnet, daß** die Mikroemulsion eine stabile und transparente-Emulsion ist, deren disperse Phase eine mittlere Teilchengröße von kleiner 100 nm aufweist.

11. Mikroemulsion gemäß, einem der vorhergehenden Ansprüche, **dadurch gekennzeichnet, daß** die Mikroemulsion kleiner 0,5 Gew.-% anionische Tenside des Sulphonät-Typs enthält und insbesondere kleiner 0,5 Gew.-% Fettsäurepolyglykolestersulfate, vorzugsweise keine Fettsäurepolyglykolestersulfate.

12. Verwendung der Mikroemulsion gemäß einem der vorhergehenden Ansprüche im kosmetischen Bereich und/oder zur Herstellung eines Arzneimittels zur Verwendung im medizinisch-dermatologischen Bereich.

13. Verwendung der Mikroemulsion gemäß einem der Ansprüche 1 bis 11 enthaltend die Komponente (E.1) als Sonnenschutzmittel.

14. Verwendung gemäß Anspruch 13 als Mittel zur Reinigung und Pflege der Haut in Form eines Schaumes zur Aufbringung mittels handbetriebener Pumpschäumer ohne Verwendung von Treibgasen

15. Verwendung gemäß Anspruch 13 oder 14 als aufzutragendes und mit wasser abzuspülendes Reinigungs- und Pflegeprodukt enthaltend größer 0 Gew.-% der Komponente (F).

16. Verwindung gemäß Anspruch 13 bis 15 als aufzutragendes und mit wasser abzuspülendes Reinigungs- und Pflegeprodukt für Haut und Haare, insbesondere als Duschgel.

17. Verwendung der Mikroemulsion gemäß einem der Ansprüche 1 bis 11 enthaltend die Komponente (E.2) als Antischuppenshampoo, vorzugsweise weiterhin enthaltend größer 0 Ges.-% der Komponente (F).

18. Verwendung der Mikroemulsion gemäß Anspruch 17, **dadurch gekennzeichnet, dass** als Komponente (E.2) 3-Aminopyridin (Niacinamid) und/oder 1-(4-Chlorophenoxy-1-(1-H-imidazol-1-yl)-3,3-dimethyl-2-butanon (Climbazol) eingesetzt wird.

19. Mikroemulsion gemäß Anspruch 4, **dadurch gekennzeichnet, dass** als Komponente (E.2) 3-Aminopyridin (Niacinamid) und/oder 1-(4-Chlorophenoxy-1-(1-H-imidazol-1-yl)-3,3-dimethyl-2-butanon (Climbazol) eingesetzt wird.

## Claims

1. A microemulsion comprising at least the following components:
(A) 0.5 to 70 % by weight of alkanolammonium salts of the alkylsulfates and/or alkyl polyalkylene glycol ether sulfates having the following structure
R¹-O-(CₚH₂ₚO)ₘ-SO₃-HN⁺R²R³R⁴,
wherein
R¹ = is a C₈- to C₂₀-hydrocarbon residue,
p = is an integer from 2 to 5, where p can be different for each m,
R² = is H, a C₁- to C₆-alkyl or a C₂- to C₄-hydroxyalkyl,
R³ = is H, a C₁- to C₆-alkyl or a C₂- to C₄-hydroxyalkyl,
R⁴ = is a C₂- to C₄-hydroxyalkyl, and
m = is an integer from 0 to 7,
or mixtures thereof,
(B) 20 to 95 % by weight of water,
(C) 0.1 to 20 % by weight of one or more oil component(s), and
(D) 0.1 to 20 % by weight of one or more mono- or polyhydroxy C₂- to C₂₄-alcohol(s), as well as
(E.1) 0.1 to 15 % by weight of one or more UV filter(s) and/or
(E.2) 0.1 to 3 % by weight of one or more antidandruff substance(s),
each percentage based on the total composition.

2. A microemulsion according to claim 1, **characterized in that** the UV filter(s) is (are) chosen from among the group of 3-benzylidenecamphor and its derivatives, 4-aminobenzoic acid derivatives, cinnamic acid esters, salicylic acid esters, benzophenone derivatives, benzalmalonic acid esters, triazine derivatives, propane-1,3-diones, phenylbenzimidazolsulfonic acid and the salts thereof, sulfonic acid derivatives of benzophenone, sulfonic acid derivatives of 3-benzylidene camphor, 4-aminobenzoic acid derivatives, and finely dispersed metal oxides or salts.

3. A microemulsion according to any one of the preceding claims, **characterized in that** the UV filter (E.1) is one or more of the following substances: octocrylenes, 4-methoxy cinnamic acid-2-ethylhexyl ester, 2-phenyl benzimidazol-5-sulfonic acid, 2-hydroxy-4-methoxy benzophenone sulfonic acid, and 4-bis(polyethoxy)para-aminobenzoic acid polyethoxy ethyl ester, and mixtures thereof.

4. A microemulsion according to claim 1, **characterized in that** the antidandruff substance (E.2) is one or more of the following substances: 1-(4-chloro-phenoxy-1-(1-H-imidazol-1-yl)-3,3-dimethyl-2-butanone, 3-amino pyridine, and the compound composed of 2-aminoethanol and 1-hydroxy-4-methyl-6-(2,4,4-trimethylpentyl)-2(1H)-pyridone.

5. A microemulsion according to claim 1, wherin the alkanol ammonium salts of the alkylsulfates and/or alkylpolyalkylene glycol ethersulfates have independently of one another the following residues or indices:
R¹ = a linear or saturated C₁₂- to C₁₆-alkyl residue,
p = 2 or 3, where p may be different for each m,
R² = H or hydroxyisopropyl,
R³ = H or hydroxyisopropyl,
R⁴ = hydroxyisopropyl and
m = is an integer from 0 to 2.

6. A microemulsion according to any one of the preceding claims, wherein the components are contained in the microemulsion:
2 to 60 % by weight of component (A)
30 to 80 % by weight of component (B)
0.5 to 15% by weight of component (C) and
0.1 to 9 % by weight of component (D).

7. A microemulsion according to any one of the preceding claims, wherein the microemulsion further comprises at least one of the following components:
(F) greater than 0 to 20 % by weight of one or more additional surfactant(s) or emulsifier(s),
and optionally also
(G) greater than 0 to 20 % by weight of one or more electrolyte(s), and
(H) greater than 0 to 10 % by weight of one or more additive(s).

8. A microemulsion according to claim 7 comprising at least the following component:
(F) at least 1 % by weight of a product from alkoxylation of triglycerides, which is esterified, wholly or in part, with C₆- to C₂₂-fatty acids, wherein preferably 2 to 40 moles of alkoxylation agent are used per mole of triglyceride.

9. A microemulsion according to any one of the preceding claims, wherein the oil component (C) contains one or more components chosen from the group of lecithins, mono-, di-, and/or triglycerides of saturated and/or unsaturated, branched and/or linear carboxylic acids having a chain length from 8 to 24 carbon atoms, branched and/or linear hydrocarbons, waxes, Vaseline, paraffin oils, polyolefins, silicone oils, and esters of saturated, unsaturated and/or aromatic, branched and/or linear carboxylic acids having a chain length from 3 to 30 carbon atoms and saturated and/or unsaturated, branched and/or linear alcohols having a chain length from 3 to 30 carbon atoms.

10. A microemulsion according to any one of the preceding claims, **characterized in that** the microemulsion is a stable and transparent emulsion with an average particle size of less than 100 nm in its disperse phase.

11. A microemulsion according to any one of the preceding claims, **characterized in that** the microemulsion contains less than 0.5 % by weight of anionic surfactants of the sulfonate type and particularly less than 0.5 % by weight of fatty acid polyglycol ester sulfates, preferably no fatty acid polyglycol ester sulfates.

12. The use of the microemulsion according to any one of the preceding claims in cosmetic applications and/or the production of a medical product for medicinal-dermatologic applications.

13. The use of a the microemulsion according to any one of claims 1 to 11, wherein the emulsion contains the component (E.1) as a sunscreen protectant.

14. The use according to claim 13 as a preparation for cleaning and treating the skin in the form of a foam applied by means of manually operated pump for dispensing foam without using propellants.

15. The use according to claim 13 or 14 as a cleaning and treating preparation, than can be applied and rinsed off with water comprising more than 0 % by weight of the component (F).

16. The use according to claim 13 to 15 as a cleaning and treating preparation, than can be applied and rinsed off with water for the skin and hair, particularly as a shower gel.

17. The use of the microemulsion according to any one of claims 1 to 11 as an antidandruff shampoo, which contains the component (E.2), and preferably also contains more than 0 % by weight of the component (F).

18. The use of the microemulsion according to any claim 17 charaterized in that as component (E.2) 3-amino pyridine (Niacinamid) and/or 1-(4-chloro-phenoxy-1-(1-H-imidazol-1-yl)-3,3-dimethyl-2-butanone (Climbazol) is used.

19. A microemulsion according to claim 4, **characterized in that** as component (E.2) 3-amino pyridine (Niacinamid) and/or 1-(4-chlorophenoxy-1-(1-H-imidazol-1-yl)-3,3-dimethyl-2-butanone (Climbazole) is used.

## Revendications

1. Microémulsion contenant au moins les composants suivants :
(A) 0,5 à 70 % en poids de sels d'alcanolammonium d'alkyl sulfates et/ou d'alkyl polyalkylène glycol éther sulfates de structure :
R¹-O-(CₚH₂ₚO)ₘ-SO₃⁻HN⁺R²R³R⁴,
dans laquelle
R¹ = un radical hydrocarbure en C₈ à C₂₀,
p = un nombre entier de 2 à 5, où p peut être différent pour chaque m,
R² = H, un alkyle en C₁ à C₆ ou un hydroxyalkyle en C₂ à C₄,
R³ = H, un alkyle en C₁ à C₆ ou un hydroxyalkyle en C₂ à C₄,
R⁴ = un hydroxyalkyle en C₂ à C₄ et
m = un nombre entier de 0 à 7,
ou leurs mélanges,
(B) 20 à 95 % en poids d'eau,
(C) 0,1 à 20 % en poids d'un ou de plusieurs composants huileux et
(D) 0,1 à 20 % en poids d'un ou de plusieurs alcools en C₂ à C₂₄ mono- ou multivalents, ainsi que
(E.1) 0,1 à 15 % en poids d'un ou de plusieurs filtres photoprotecteurs anti-UV et/ou
(E.2) 0,1 à 3 % en poids d'un ou de plusieurs agents antipelliculaires,
à chaque fois par rapport à la composition totale.

2. Microémulsion selon la revendication 1, **caractérisée en ce que** le ou les filtres photoprotecteurs anti-UV sont choisis dans le groupe : 3-benzylidène camphre et ses dérivés, dérivés d'acide 4-aminobenzoïque, esters d'acide cinnamique, esters d'acide salicylique, dérivés de benzophénone, esters d'acide benzalmalonique, dérivés de triazine, propane-1,3-dione, acide phénylbenzimidazolsulfonique et ses sels, dérivés d'acide sulfonique de benzophénone, dérivés d'acide sulfonique du 3-benzylidène camphre, dérivés de l'acide 4-aminobenzoïque et d'oxydes métalliques finement dispersés ou leurs sels.

3. Microémulsion selon l'une des revendications précédentes, **caractérisée en ce que** le filtre photoprotecteur anti-UV (E.1) est une ou plusieurs des substances suivantes : octocrylène, ester 2-éthylhexylique d'acide 4-méthoxycinnamique, acide 2-phénylbenzimidazol-5-sulfonique, acide 2-hydroxy-4-méthoxybenzophénone-sulfonique et ester polyéthoxyéthylique d'acide 4-bis(polyéthoxy)para-aminobenzoïque ainsi que leurs mélanges.

4. Microémulsion selon la revendication 1, **caractérisée en ce que** l'agent antipelliculaire (E.2) est une ou plusieurs des substances suivantes : 1-(4-chlorophénoxy)-1-(1H-imidazol-1-yl)-3,3-diméthyl-2-butanone, 3-aminopyridine et le composé du 2-aminoéthanol avec de la 1-hydroxy-4-méthyl-6-(2,4,4-triméthylpentyl)-2(1H)-pyridone.

5. Microémulsion selon la revendication 1, dans laquelle les sels d'alcanolammonium d'alkyl sulfates et/ou d'alkyl polyalkylène glycol éther sulfates présentent, indépendamment les uns des autres, les radicaux ou indices suivants :
R¹ = un radical alkyle en C₁₂ à C₁₆ linéaire ou saturé,
p = 2 ou 3, où p peut être différent pour chaque m,
R² = H ou hydroxyisopropyle,
R³ = H ou hydroxyisopropyle,
R⁴ = hydroxyisopropyle et
m = un nombre entier de 0 à 2,

6. Microémulsion selon l'une des revendications précédentes, dans laquelle les composants
(A) à un taux de 2 à 60 % en poids,
(B) à un taux de 30 à 80 % en poids,
(C) à un taux de 0,5 à 15 % en poids et
(D) à un taux de 0,1 à 9 % en poids
sont contenus dans la microémulsion.

7. Microémulsion selon l'une des revendications précédentes, contenant en outre au moins un des composants suivants :
(F) plus de 0 à 20 % en poids d'un ou de plusieurs autres tensioactifs ou émulsifiants,
et le cas échéant aussi
(G) plus de 0 à 20 % en poids d'un ou de plusieurs électrolytes et
(F) plus de 0 à 10 % en poids d'un ou de plusieurs additifs.

8. Microémulsion selon la revendication 7, contenant au moins les composants suivants :
(F) au moins 1 % en poids d'un produit issu de l'alcoxylation des triglycérides, qui est estérifié totalement ou partiellement avec des acides gras en C₆ à C₂₂, où l'on utilise de préférence par mole de triglycéride 2 à 40 moles d'agents alcoxylants.

9. Microémulsion selon l'une des revendications précédentes, dans laquelle le composant huileux (C) contient un ou plusieurs composants choisis dans le groupe constitué par les lécithines, les mono-, di- et/ou triglycérides d'acides carboxyliques saturés et/ou insaturés, ramifiés et/ou linéaires d'une longueur de chaîne de 8 à 24 atomes de C, les hydrocarbures ramifiés et/ou linéaires, les cires ; les vaselines, les huiles de paraffine, les polyoléfines, les huiles siliconées et les esters d'acides carboxyliques saturés, insaturés, et/ou aromatiques, ramifiés et/ou linéaires d'une longueur de chaîne de 3 à 30 atomes de C et les alcools saturés et/ou insaturés, ramifiés et/ou linéaires d'une longueur de chaîne de 3 à 30 atomes de C.

10. Microémulsion selon l'une des revendications précédentes, **caractérisée en ce que** la microémulsion est une émulsion stable et transparente, dont la phase dispersée présente une granulométrie moyenne de moins de 100 nm.

11. Microémulsion selon l'une des revendications précédentes, **caractérisée en ce que** la microémulsion contient moins de 0,5 % en poids de tensioactifs anioniques du type sulfonate et en particulier moins de 0,5 % en poids de sulfates d'esters de polyglycol d'acides gras, de préférence aucun sulfate d'esters de polyglycol d'acides gras.

12. Utilisation de la microémulsion selon l'une des revendications précédentes dans le domaine cosmétique et/ou pour la fabrication d'un médicament destiné à l'utilisation dans le domaine médico-dermatologique.

13. Utilisation de la microémulsion selon l'une des revendications 1 à 11, contenant le composant (E.1) à titre d'agent antisolaire.

14. Utilisation selon la revendication 13 comme produit de nettoyage et de soin de la peau sous la forme d'une mousse pour l'application au moyen d'un générateur de mousse à pompe actionné manuellement sans utilisation de gaz propulseurs.

15. Utilisation selon la revendication 13 ou 14 comme produit de nettoyage et de soin à appliquer et à rincer à l'eau, contenant plus de 0 % en poids du composant (F).

16. Utilisation selon les revendications 13 à 15 comme produit de nettoyage et de soin pour la peau et les cheveux à appliquer et à rincer à l'eau, en particulier comme gel douche.

17. Utilisation de la microémulsion selon l'une des revendications 1 à 11 contenant le composant (E.2) comme shampooing antipelliculaire, de préférence contenant également plus de 0 % en poids du composant (F).

18. Utilisation de la microémulsion selon la revendication 17, **caractérisée en ce que** l'on utilise comme composant (E.2) de la 3-aminopyridine (niacinamide) et/ou de la 1-(4-chlorophénoxy)-1-(1H-imidazol-1-yl)-3,3-diméthyl-2-butanone (climbazol).

19. Microémulsion selon la revendication 4, **caractérisée en ce que** l'on utilise comme composant (E.2) de la 3-aminopyridine (niacinamide) et/ou de la 1-(4-chlorophénoxy)-1-(1H-imidazol-1-yl)-3,3-diméthyl-2-butanone (climbazol).
